# EUROPEAN PATENT APPLICATION

(11) **EP 4 431 944 A1**
(43) Date of publication of application: **18.09.2024**
(21) Application number: 22892259.7
(22) Date of filing: 14.11.2022
(51) Int. Cl.: G01N 33/68, G01N 33/574, C12Q 1/6886, C12Q 1/6883

(54) **BIOMARKER FOR REGULATORY T CELL**

(30) Priority: 12.11.2021 US 202163278610 P
(71) Applicant: JRD Science, Inc., Los Angeles, CA 90024 (US)
(72) Inventor: LEE, Christina Rae-Kyung, Los Angeles, California 90024 (US)
(74) Representative: Müller, Christian Stefan Gerd
(86) International application number: PCT/IB2022/060908
(87) International publication number: WO 2023/084474

(57) **Abstract**

The present invention relates to a biomarker for regulatory T cells.

## Description

### Technical Field

Biomarkers for regulatory T cells.

### Background Art

It has been reported that regulatory T cells (Tregs) play an important role in naturally preventing the occurrence of excessive inflammation and immune responses, but when autoimmune diseases and chronic inflammatory diseases occur, the function and number of regulatory T cells remarkably decrease. Thus, for patients with immune diseases and inflammatory diseases, it is important that regulatory T cells are generated at normal levels, which can be one of the methods for treatment of these diseases.

It has been reported that, in the case of cancers, regulatory T cells are widely distributed in the cancer microenvironment and suppress the cancer cell killing ability of natural killer cells (NK cells) or cancer-specific cytotoxic T lymphocytes, and thus play an important role in cancer immune evasion mechanisms along with immune checkpoints such as PD-1 and CTLA-4.

Regulatory T cells may be classified based on their developmental origin and cell characteristics into naive regulatory T cells (naive Tregs; nTregs), resting regulatory T cells (resting Tregs; rTregs), effector regulatory T cells (effector Tregs; eTregs), and memory regulatory T cells (memory Tregs; mTregs). Recently, Shimon Sakaguchi's group has classified Tregs into non-Tregs, nTregs/rTregs, and eTregs, based on the expression levels of CD4, CD45RA, Foxp3, and CD25 (CD4+CD25+CD45RA-Foxp3lo; non-Tregs, CD4+CD25+CD45RA+Foxp3lo; nTregs, and CD4+CD25+CD45RA-Foxp3hi; eTregs). Among the types of regulatory T cells described above, effector regulatory T cells and memory regulatory T cells are known to have high immunosuppressive capacity. In particular, it has been reported that effector regulatory T cells are abundant in tumor-infiltrating lymphocytes (TILs), highly express the immune checkpoint CTLA-4, an immune checkpoint, and thus have high immunosuppressive capacity. Therefore, specifically regulating effector regulatory T cells, memory regulatory T cells, and regulatory T cells in tumor-infiltrating lymphocytes, which have high immunosuppressive capacity, among regulatory T cells, may be considered an effective immunotherapy method.

To date, studies have been conducted on genes and proteins present specifically in regulatory T cells, suggesting that substances such as CD25, CTLA4, CD62L, CD38, CD103, GITR, and CD45RB may correspond to marker substances. However, genes or proteins that can target regulatory T cells alone have not yet been reported.

### DISCLOSURE

### Technical Problem

An object of the present invention is to provide a method of identifying regulatory T cells using an antibody or antigen-binding fragment that binds specifically to a marker or a fragment thereof specific for regulatory T cells.

Another object of the present invention is to provide a composition for diagnosing cancer, immune-related disease, or brain nervous system disease, comprising an agent for measuring the expression level of a marker or a fragment thereof specific for regulatory T cells, or a gene encoding the marker or fragment.

Still another object of the present invention is to provide a diagnostic kit comprising a composition for diagnosing cancer, immune-related disease, or brain nervous system disease, the composition comprising an agent for measuring the expression level of a marker or a fragment thereof specific for regulatory T cells, or a gene encoding the marker or fragment.

Yet another object of the present invention is to provide a method of providing information for diagnosing cancer, immune-related disease, or brain nervous system disease, comprising a step of measuring the expression of a marker or a fragment thereof specific for regulatory T cells, or a gene encoding the marker or fragment, in a biological sample isolated from a subject of interest.

However, objects to be achieved by the present invention are not limited to the problems mentioned above, and other objects not mentioned above will be clearly understood by those skilled in the art from the following description.

### Technical Solution

"Regulatory T cells" or "Tregs" of the present invention may be selected from the group consisting of effector regulatory T cells, memory regulatory T cells, and regulatory T cells in tumor-infiltrating lymphocyte, which have high immunosuppressive capacity, without being limited thereto.

"Nucleic acid molecules" of the present invention include any nucleic acid molecules having polynucleotide sequences which are translated into the amino acid sequences of the polypeptides provided by the present invention as known to those skilled in the art. Therefore, various polynucleotide sequences with open reading frames (ORFs) may be produced, which are also all included in the nucleic acid molecules of the present invention.

In the present invention, the term "antibody" refers to a substance that binds specifically to an antigen, causing an antigen-antibody reaction. For the purposes of the present invention, the term "antibody" refers to an antibody that binds specifically to a protein or an extracellular domain thereof specific for regulatory T cells. Examples of the antibody of the present invention include all of polyclonal antibodies, monoclonal antibodies, and recombinant antibodies. The antibodies may be readily produced using techniques well known in the art. For example, a polyclonal antibody may be produced by a method well known in the art, which includes a process of obtaining a serum containing the antibody by injecting the antigen of the biomarker protein into an animal and collecting blood from the animal. This polyclonal antibody may be produced from any animal such as goat, rabbit, sheep, monkey, horse, pig, cow, dog, or the like. In addition, a monoclonal antibody may be produced using the hybridoma method well known in the art (see Kohler and Milstein (1976) European Journal of Immunology 6:511-519), or the phage antibody library technology (see Clackson et al, Nature, 352:624-628, 1991; Marks et al, J. Mol. Biol., 222:58, 1-597, 1991). The antibody produced by the above method may be isolated and purified using a method such as gel electrophoresis, dialysis, salt precipitation, ion exchange chromatography, or affinity chromatography. In addition, examples of the antibody of the present invention include not only a complete form having two full-length light chains and two full-length heavy chains, but also functional fragments of an antibody molecule. "Functional fragment of an antibody molecule" refers to a fragment having at least an antigen-binding function, and examples thereof include Fab, F(ab'), F(ab')2, and Fv.

In the present invention, the "immunoglobulin" has a heavy chain and a light chain, and each of the heavy chain and the light chain comprises a constant region and a variable region. The variable region of each of the light chain and the heavy chain contains three hypervariable regions, called complementarity determining regions (hereinafter referred to as "CDRs"), and four framework regions. The CDRs primarily serve to bind to an antigenic determinant (epitope) of an antigen. The CDRs of each chain are typically referred to as CDR1, CDR2, and CDR3, numbered sequentially starting from the N-terminus, and are also typically identified by the chain in which the particular CDR is located.

In the present invention, the variable region comprises three hypervariable regions, called complementarity determining regions (CDRs), and four framework regions. The CDRs primarily serve to bind to an antigenic determinant (epitope) of an antigen. The CDRs of each chain are typically referred to as CDR1, CDR2, and CDR3, numbered sequentially starting from the N-terminus, and are also typically identified by the chain in which the particular CDR is located.

In the present invention, the "full-length antibody" has a structure having two full-length light chains and two full-length heavy chains, wherein each of the light chains is linked to the heavy chain via a disulfide bond. Examples of the full-length antibody include IgA, IgD, IgE, IgM, and IgG. Subtypes of the IgG include IgG1, IgG2, IgG3, and IgG4.

In the present invention, the term "antigen-binding fragment" refers to a fragment having an antigen-binding function. Examples of the antigen-binding fragment include: (i) a Fab fragment consisting of a light chain variable region (VL), a heavy chain variable region (VH), a light chain constant region (CL), and a heavy chain constant region 1 (CH1); (ii) a Fd fragment consisting of VH and CH1 domains; (iii) a Fv fragment consisting of VL and VH domains of a single antibody; (iv) a dAb fragment consisting of a VH domain; (v) an isolated CDR region; (vi) a F(ab')₂ fragment which is a bivalent fragment including two linked Fab fragments; (vii) a single-chain Fv molecule

(scFv) in which a VH domain and a VL domain are linked together by a peptide linker that allows the two domains to associate to form an antigen-binding site; (viii) a bispecific single-chain Fv dimer; and (ix) a diabody which is a multivalent or multispecific fragment constructed by gene fusion. The antigen-binding fragment may be obtained as a Fab or F(ab')2 fragment when a proteolytic enzyme, for example, papain or pepsin, is used, and the antigen-binding fragment may also be produced through a genetic recombination technique.

In the present invention, the term "monoclonal antibody" refers to an antibody molecule having a single molecular composition, which is obtained from a population of substantially identical antibodies. The monoclonal antibody exhibits single binding specificity and affinity for a particular epitope.

In the present invention, the term "binding" or "specific binding" refers to the affinity of the antibody or antibody composition of the present invention for an antigen. In antigen-antibody binding, the "specific binding" is distinguishable from non-specific background binding, typically when the dissociation constant (Kd) is less than 1×10⁻⁵ M, less than 1×10⁻⁶M, or less than 1×10⁻⁷ M. Specific binding can be detected by methods known in the art, such as ELISA, surface plasmon resonance (SPR), immunoprecipitation, and coprecipitation, which include an appropriate control that can distinguish between non-specific binding and specific binding.

The antibody or antigen-binding fragment of the present invention may exist as a multimer, such as a dimer, a trimer, a tetramer, or a pentamer, which includes at least a portion of the antigen-binding activity of a monomer. Such multimers also include a homomultimer or a heteromultimer. Antibody multimers contain a large number of antigen-binding sites, and thus have excellent antigen-binding activity compared to monomers. Antibody multimers are also easily used to produce multifunctional (bifunctional, trifunctional or tetrafunctional) antibodies.

In the present invention, "prevention" may include, without limitation, any action that blocks the symptoms of a disease or suppresses or delays the symptoms of the disease by using the pharmaceutical composition of the present invention.

In the present invention, "treatment" and "amelioration" may include, without limitation, any action that alleviates or beneficially alters symptoms of a disease by using the pharmaceutical composition of the present invention.

In the present invention, for use, the pharmaceutical composition may be formulated in the form of oral preparations such as powders, granules, capsules, tablets, and aqueous suspensions, preparations for external use, suppositories, and sterile injectable solutions, according to the respective conventional methods, without being limited thereto. The pharmaceutical composition of the present invention may further contain pharmaceutically acceptable carriers. As the pharmaceutically acceptable carriers, a binder, a lubricant, a disintegrant, an excipient, a solubilizer, a dispersant, a stabilizer, a suspending agent, a colorant, a flavoring agent, and the like may be used for oral administration; a buffer, a preserving agent, a pain-relieving agent, a solubilizer, an isotonic agent, a stabilizer, and the like may be used for injection; and a base, an excipient, a lubricant, a preservative, and the like may be used for topical administration. The pharmaceutical composition of the present invention may be prepared in various dosage forms by being mixed with the pharmaceutically acceptable carriers as described above. For example, for oral administration, the pharmaceutical composition may be formulated in the form of tablets, troches, capsules, elixirs, suspensions, syrups, wafers, or the like. For injection, the pharmaceutical composition may be formulated in the form of unit dosage ampoules or in multiple-dosage forms. In addition, the pharmaceutical composition may be formulated into solutions, suspensions, tablets, capsules, sustained-release preparations, or the like. Meanwhile, examples of carriers, diluents or excipients suitable for formulation include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, and mineral oil. In addition, a filler, an anti-coagulant, a lubricant, a wetting agent, a fragrance, an emulsifier, a preservative, and the like may further be contained.

In the present invention, the routes of administration of the pharmaceutical composition of the present invention include oral, intravenous, intramuscular, intraarterial, intramedullary, intradural, intracardiac, transdermal, subcutaneous, intraperitoneal, intranasal, intestinal, topical, sublingual, or rectal routes, without being limited thereto. Oral or parenteral administration is preferred.

In the present invention, the "parenteral" includes subcutaneous, intradermal, intravenous, intramuscular, intra-articular, intra-synovial, intrasternal, intradural, intralesional and intra-cranial injection or infusion techniques. The pharmaceutical composition of the present invention may also be formulated as suppositories for intrarectal administration.

The "dosage regimen" of the pharmaceutical composition of the present invention may vary depending on a variety of factors, including the activity of a specific compound used, the patient's age, body weight, general health status, sex and diet, the time of administration, the route of administration, the rate of excretion, drug combination, and the severity of a specific disease to be prevented or treated. Although the dosage of the pharmaceutical composition may vary depending on the patient's condition and body weight, the severity of disease, the drug form, and the route and duration of administration, it may be appropriately selected by those skilled in the art, and the pharmaceutical composition may be administered at a dose of 0.0001 to 50 mg/kg/day or 0.001 to 50 mg/kg/day. This administration may be done once a day or several times a day. The dose is not intended to limit the scope of the present invention in any way. The pharmaceutical composition according to the present invention may be formulated in the form of pills, sugar-coated tablets, capsules, liquids, gels, syrups, slurries, or suspensions.

In the present invention, "PNA (peptide nucleic acid)" refers to an artificially synthesized DNA or RNA-like polymer, which was first introduced by the Professors Nielsen, Egholm, Berg and Buchardt at University of Copenhagen, Denmark in 1991. DNA has a phosphate-ribose sugar backbone, but PNA has repeated N-(2-aminoethyl)-glycine backbones linked via peptide bonds, and thus has a significantly increased binding affinity for DNA or RNA and significantly increased stability. Thus, PNA is used for molecular biology, diagnostic assays and antisense therapies. The PNA is disclosed in detail in the literature [Nielsen P E, Egholm M, Berg R H, Buchardt O (December 1991). "Sequence-selective recognition of DNA by strand displacement with a thymine-substituted polyamide". Science 254(5037): 1497-1500].

In the present invention, the "aptamer" refers to an oligonucleotide or a peptide molecule, and the general contents of the aptamer are disclosed in detail in the literature [Bock L C et al., Nature 355(6360):5646(1992); Hoppe-Seyler F, Butz K "Peptide aptamers: powerful new tools for molecular medicine". J Mol Med. 78(8):42630(2000); Cohen B A, Colas P, Brent R. "An artificial cell-cycle inhibitor isolated from a combinatorial library". Proc Natl Acad Sci USA. 95(24): 142727(1998)].

In the present invention, the term "primer" refers to a fragment that recognizes a target gene sequence, and comprises a pair of forward and reverse primers, but is preferably a pair of primers providing analysis results with specificity and sensitivity. When the nucleic acid sequence of the primer is a sequence inconsistent with the non-target sequence present in the sample, and thus is a primer that amplifies only the target gene sequence containing the complementary primer binding site without inducing non-specific amplification, high specificity may be imparted.

In the present invention, the term "probe" refers to a substance capable of binding specifically to a target substance to be detected in a sample, and refers to a substance capable of specifically detecting the presence of the target substance in the sample through the binding. The type of probe is not particularly limited so long as it is commonly used in the art. Preferably, the probe may be PNA (peptide nucleic acid), LNA (locked nucleic acid), a peptide, a polypeptide, a protein, an RNA, or a DNA, and is most preferably PNA. More specifically, the probe is a biomolecule derived from an organism or an analogue thereof, or is produced *in vitro.* Examples of the probe include an enzyme, a protein, an antibody, a microorganism, an animal and/or plant cell and organ, a neuron, DNA, and RNA. Examples of the DNA include cDNA, genomic DNA, and an oligonucleotide, examples of the RNA include genomic RNA, mRNA and an oligonucleotide, and examples of the protein include antibodies, antigens, enzymes, peptides, and the like.

In the present invention, the term "LNA (locked nucleic acid)" refers to a nucleic acid analogue containing a 2'-O or 4'-C methylene bridge. LNA nucleosides comprise the common nucleobases of DNA and RNA, and can form base pairs according to the Watson-Crick base-pair rule. However, LNA fails to form an ideal shape in the Watson-Crick bond due to "locking" of the molecule attributable to the methylene bridge. When LNA is incorporated in a DNA or RNA oligonucleotide, it can more rapidly pair with a complementary nucleotide chain, thus increasing the stability of the double strand.

In the present invention, the term "antisense" means an oligomer that has a nucleotide sequence and a backbone between subunits, wherein an antisense oligomer is hybridized with the target sequence in the RNA by Watson-Crick base pairing to typically allow the formation of the mRNA and RNA:oligomer heterodimers in the target sequence. The oligomer may have an accurate or approximate sequence complementarity to the target sequence.

The kit of the present invention may be an RT-PCR kit, a DNA chip kit, an ELISA kit, a protein chip kit, a rapid kit, or a multiple-reaction monitoring (MRM) kit, without being limited thereto. The diagnostic kit may further comprise one or more other component compositions, solutions or devices suitable for the assay method. For example, the diagnostic kit may further comprise essential elements necessary for carrying out a reverse transcription polymerase reaction. The reverse-transcription polymerase chain reaction kit comprises a pair of primers specific for the gene encoding the marker protein. The primer is an oligonucleotide having a sequence specific to the nucleic acid sequence of the gene and may have a length of about 7 bp to 50 bp, more preferably about 10 bp to 30 bp. The kit may also comprise a primer specific to the nucleic acid sequence of a control gene. In addition, the reverse-transcription polymerase chain reaction kit may comprise a test tube or other suitable container, a reaction buffer (various pHs and magnesium concentrations), deoxynucleotides (dNTPs), enzymes such as Taq-polymerase and reverse transcriptase, DNAse, an RNAse inhibitor, DEPC-water, sterilized water, etc. The diagnostic kit may comprise essential elements required to perform DNA chip assay. The DNA chip kit may comprise a substrate having immobilized thereon a cDNA or oligonucleotide corresponding to the gene or a fragment thereof, a reagent for constructing a fluorescence-labeled probe, an agent, an enzyme, and the like. In addition, the substrate may comprise a cDNA or oligonucleotide corresponding to a control gene or a fragment thereof. The diagnostic kit of the present invention may comprise essential elements required to perform ELISA. The ELISA kit comprises an antibody specific to the protein. The antibody has a high specificity and affinity for the marker protein and exhibits little or no cross-reactivity with other proteins. It is a monoclonal antibody, a polyclonal antibody or a recombinant antibody. The ELISA kit may also comprise an antibody specific to a control protein. In addition, the ELISA kit may comprise reagents capable of detecting bound antibodies, for example, labelled secondary antibodies, chromophores, enzymes (e.g., conjugated with antibodies), and substrates thereof or other substances capable of binding to antibodies.

In the present invention, the term "subject of interest" refers to an individual whose disease onset is uncertain and who is highly likely to develop the disease.

In the present invention, the term "biological sample" refers to any material, biological fluid, tissue or cell obtained from or derived from a subject. For example, the biological sample may contain whole blood, leukocytes, peripheral blood mononuclear cells, buffy coat, plasma, serum, sputum, tears, mucus, nasal washes, nasal aspirate, breath, urine, semen, saliva, peritoneal washings, pelvic fluids, cystic fluid, meningeal fluid, amniotic fluid, glandular fluid, pancreatic fluid, lymph fluid, pleural fluid, nipple aspirate, bronchial aspirate, synovial fluid, joint aspirate, organ secretions, cells, cell extract, or cerebrospinal fluid, without being limited thereto.

In the present disclosure, "candidate substance" comprises any substance, molecule, element, compound, entity, or a combination thereof. Examples of the candidate substance include, but are not limited to, proteins, polypeptides, small organic molecules, polysaccharides, polynucleotides, and the like. In addition, the candidate substance may also be a natural product, a synthetic compound, or a combination of two or more substances.

It is possible to use searching for phage-displayed peptide clones that bind to the protein, high-throughput screening (HTS) using natural material and chemical libraries, drug hit HTS, cell-based screening, or DNA array-based screening. In this case, the composition of the present invention may comprise, in addition to the protein, a buffer or reaction solution to stably maintain the structure or physiological activity of the protein. In addition, the composition of the present invention may comprise, for *in vivo* experiments, cells expressing the protein, or cells containing a plasmid expressing the protein under a promoter capable of regulating the transcription rate. In the screening method of the present invention, test substances include individual nucleic acids, proteins, other extracts or natural products, compounds, etc., which are assumed to have a potential as a cancer metastasis inhibitor according to the conventional screening manner or are randomly selected. A test substance exhibiting an activity of enhancing the expression of gene or enhancing the function of the protein, obtained through the screening method of the present invention, may provide an immunosuppressive candidate by developing an inhibitor against the test substance, and conversely, a test substance exhibiting an activity of inhibiting the expression of the gene or inhibiting the function of the protein, obtained through the screening method of the present invention, may serve as an immunosuppressive candidate. Such an immunosuppressive candidate may act as a leading compound in the subsequent process of developing an immunosuppressive agent, and it is possible to develop a new immunosuppressive agent by modifying and optimizing the structure of the leading compound so that the leading compound is able to exhibit the effect of inhibiting the function of the gene or the protein expressed therefrom.

In the present invention, the term "small interfering RNA (siRNA)" refers to a short (19 to 30 bp) double-strand RNA duplex which, when introduced into a cell, exhibits the effect of inhibiting only the expression of a specific gene without non-specific inhibition. In the known mechanism of action of siRNA, siRNA binds to the RNA-induced silencing complex (RISC) in the cell, the sense strand of the gene corresponding to the mRNA is cut out, and the antisense stand, which is complementary to the sense strand, is in a complex with the RISC and binds to and degrades the mRNA having a complementary nucleotide sequence, that is, the mRNA of the target gene, thereby inhibiting the expression of the gene.

In the present invention, the term "short hairpin RNA (shRNA)" refers to one obtained by a process in which an oligo DNA comprising a 3- to 10-base linker located between a sense strand of the nucleotide sequence of the target gene siRNA and an antisense strand complementary thereto is synthesized and then cloned into a plasmid vector. When shRNA is inserted into or expressed in retrovirus such as lentivirus or adenovirus, shRNA with a looped hairpin structure is produced and processed into siRNA by Dicer in the cell, thereby exhibiting RNAi effects. shRNA has the advantage of exhibiting RNAi effects for a relatively long period of time. The siRNA of the present invention may be in a chemically modified form to prevent rapid degradation by nucleases *in vivo.* Because siRNA has a double-helix structure, it is a relatively more stable structure than a ribonucleic acid or antisense oligonucleotide having a single-helix structure. However, since siRNA is quickly degraded by nucleases *in vivo,* the degradation rate thereof may be reduced through chemical modification. Methods for chemical modification of siRNA to make siRNA stable and resistant to easy degradation are well known to those skilled in the art. The most commonly used method for chemical modification of siRNA is modification with boranophosphate or phosphorothioate. These substances form a stable linkage between the nucleosides of siRNA, and consequently confer resistance to degradation by nucleases. Although ribonucleic acid modified with boranophosphate has the property of being resistant to degradation by nucleases, it is not produced through a chemical reaction and is synthesized only by a process in which boranophosphate is incorporated into ribonucleic acid through an *in vitro* transcription reaction. Although the method of modification with boranophosphate is a relatively easy method, it has a disadvantage in that modification at a specific location is difficult. On the other hand, the method of modification with phosphorothioate has an advantage in that it is possible to introduce sulfur elements into the desired portions, but a high degree of phosphothioation may cause problems such as reduced efficacy, toxicity, and non-specific formation of the RNA-induced silencing complex (RISC). Therefore, in addition to the above two methods, a method of conferring resistance to nucleases by chemically modifying only the termination site (exceeding the 3' end) of ribonucleic acid has recently been preferred. It is also known that chemical modification of the ribose ring also increases resistance to nucleases, and in particular, modification of the 2'-position of the ribose originally present in the cell stabilizes siRNA. However, stability increases only when a methyl group is introduced exactly at this position, and on the contrary, if too many methyl groups are introduced, they may cause problems such as loss of the ribonucleic acid-mediated interference phenomenon. The reason for this chemical modification is also to increase the *in vivo* pharmacokinetic residence time and increase effectiveness (Mark et. al., Molecular Therapy, 13:644-670, 2006). In addition to chemical modification methods, a safe and efficient delivery system is required to increase the intracellular delivery efficiency of siRNA. For this purpose, the siRNA of the present invention may be contained in a pharmaceutical composition for treating cancer in the form of a complex with a nucleic acid delivery system.

In the present invention, the term "cancer" refers to or describes the physiological condition in mammals that is typically characterized by unregulated cell growth. Cancer to be prevented, ameliorated or treated in the present invention may be a solid tumor consisting of an agglomerate generated by abnormal cell growth in a solid organ, and may be, without limitation, gastric cancer, liver cancer, glioblastoma, ovarian cancer, colorectal cancer, head and neck cancer, bladder cancer, renal cell cancer, breast cancer, metastatic cancer, prostate cancer, pancreatic cancer, melanoma, or lung cancer, depending on the location of the solid organ.

In the present invention, the "immune-related disease" may be, but is not limited to, at least one selected from the group consisting of autoimmune disease, graft-versus-host disease, organ transplant rejection, asthma, atopy, and acute or chronic inflammatory disease. Here, the autoimmune disease may be, but is not limited to, at least one selected from the group consisting of rheumatoid arthritis, systemic scleroderma, systemic lupus erythematosus, atopic dermatitis, psoriasis, alopecia areata, asthma, Crohn's disease, Behcet's disease, Sjogren's syndrome, Guillain-Barré syndrome, chronic thyroiditis, multiple sclerosis, multiple myositis, ankylosing spondylitis, fibrositis, and polyarteritis nodosa.

In the present invention, the "brain nervous system disease" may be a neurodegenerative disease or a neuroinflammatory disease.

In the present invention, the "neurodegenerative disease" may refer to a disease caused by a decrease or loss of function of nerve cells, and the "neuroinflammatory disease" may refer to a disease caused by an excessive inflammatory response in the nervous system.

In the present invention, a specific example of the "neurodegenerative disease" or the "neuroinflammatory disease" may be selected from the group consisting of stroke, dementia, Alzheimer's disease, Parkinson's disease, Huntington's disease, Niemann-Pick disease, multiple sclerosis, prion disease, Creutzfeldt-Jakob disease, frontotemporal dementia, dementia with Lewy bodies, amyotrophic lateral sclerosis, paraneoplastic syndrome, corticobasal degeneration, multiple system atrophy, progressive supranuclear palsy, nervous system autoimmune disease, spinocerebellar ataxia, inflammatory and neuropathic pain, cerebrovascular disease, spinal cord injury, and tauopathy, without being limited thereto.

In the present invention, the expression "method for identifying regulatory T cells" means a method of amplifying a specific marker present in regulatory T cells by reverse transcription polymerase chain reaction (RT-PCR) and identifying the specific marker present in regulatory T cells based on the fluorescence through fluorescence activated cell sorting (FACS), thereby identifying or isolating regulatory T cells, without being limited thereto.

In the present invention, the "reverse transcription polymerase reaction" is a modification of the polymerase chain reaction (PCR), which is an experimental technique commonly used in molecular biology to produce a large number of DNA sequences by an amplification process. However, in reverse transcription polymerase chain reaction, RNA is first reverse transcribed by reverse transcriptase to produce cDNA, and the produced cDNA is amplified by conventional polymerase chain reaction or real-time polymerase chain reaction, without being limited thereto.

In the present invention, the term "fluorescence activated cell sorting (FACS)" or "FACS" refers to a method of separating a population of cells into one or more sub-populations based on the presence, absence, or level of one or more FACS selectable polypeptides expressed by the cells. FACS relies on optical properties, including fluorescence, of individual cells in order to sort the cells into sub-populations.

In the present invention, the "FACS selectable polypeptide" is a polypeptide that can be detected, directly or indirectly, by flow cytometry. Examples of FACS selectable polypeptides include polypeptides that include an extracellular domain (e.g., CD52 or CD59) that are capable of being bound to a detectable binding partner (e.g., a fluorescently-labeled antibody) for indirect detection of the polypeptide by flow cytometry. Other examples of FACS selectable polypeptides include fluorescent proteins such as green fluorescent protein (GFP), red fluorescent protein (RFP), yellow fluorescent protein (YFP), blue fluorescent protein (BFP), and variants thereof including eGFP, Venus, mCherry, mTomato, and the like, which may be detected directly by flow cytometry. Through FACS, specific markers present in regulatory T cells may be identified, and regulatory T cells may be isolated.

One embodiment of the present invention provides a method of identifying regulatory T cells using an antibody or antigen-binding fragment that binds specifically to any one protein or fragment thereof selected from the group consisting of RGS1, F5, and CD27.

Another embodiment of the present invention provides the method of identifying regulatory T cells, wherein the protein is a cell surface protein that is expressed specifically on the surface of regulatory T cells.

Still another embodiment of the present invention provides the method of identifying regulatory T cells, wherein the regulatory T cells are at least one type selected from the group consisting of effector regulatory T cells, memory regulatory T cells, and regulatory T cells in tumor-infiltrating lymphocytes.

One embodiment of the present invention provides a composition for diagnosing cancer disease, comprising an agent for measuring the expression level of any one protein or fragment thereof selected from the group consisting of RGS1, F5, and CD27, or a gene encoding the protein or fragment thereof.

Another embodiment the present invention provides the composition for diagnosing, wherein the protein is a cell surface protein that is expressed specifically on the surface of regulatory T cells.

Still another embodiment of the present invention provides the composition for diagnosing, wherein the regulatory T cells are at least one type selected from the group consisting of effector regulatory T cells, memory regulatory T cells, and regulatory T cells in tumor-infiltrating lymphocytes.

Yet another embodiment of the present invention provides the composition for diagnosing, wherein the agent for measuring the expression level of the protein or fragment thereof comprises at least one selected from the group consisting of an antibody, an oligopeptide, a ligand, a peptide nucleic acid (PNA), and an aptamer, which bind specifically to the protein or fragment thereof.

Still yet another embodiment of the present invention provides the composition for diagnosing, wherein the agent for measuring the expression level of the gene encoding the protein or fragment thereof comprises at least one selected from the group consisting of a primer, a probe, and an antisense oligonucleotide, which bind specifically to the gene.

A further embodiment of the present invention provides the composition for diagnosing, wherein the cancer is ovarian cancer, colorectal cancer, pancreatic cancer, gastric cancer, liver cancer, breast cancer, cervical cancer, thyroid cancer, parathyroid cancer, lung cancer, non-small cell lung cancer, prostate cancer, gallbladder cancer, biliary tract cancer, non-Hodgkin's lymphoma, Hodgkin's lymphoma, hematological cancer, bladder cancer, kidney cancer, melanoma, colon cancer, bone cancer, skin cancer, head cancer, uterine cancer, rectal cancer, brain tumor, perianal cancer, fallopian tube carcinoma, endometrial carcinoma, vaginal cancer, vulvar carcinoma, esophageal cancer, small intestine cancer, endocrine adenocarcinoma, adrenal cancer, soft tissue sarcoma, urethral cancer, penile cancer, cancer of ureter, renal cell carcinoma, renal pelvic carcinoma, central nervous system (CNS) tumor, primary CNS lymphoma, spinal cord tumor, brainstem glioma, or pituitary adenoma.

Another embodiment of the present invention provides a kit for diagnosing cancer disease, comprising the composition for diagnosing.

One embodiment of the present invention provides a method of providing information for diagnosing cancer disease, comprising a step of measuring the expression level of any one protein or fragment thereof selected from the group consisting of RGS1, F5, and CD27, or a gene encoding the protein or fragment thereof, in a biological sample isolated from a subject of interest.

Another embodiment of the present invention provides the method of providing information, wherein the protein is a cell surface protein that is expressed specifically on the surface of regulatory T cells.

Still another embodiment of the present invention provides the method of providing information, wherein the regulatory T cells are at least one type selected from the group consisting of effector regulatory T cells, memory regulatory T cells, and regulatory T cells in tumor-infiltrating lymphocytes.

Yet another embodiment of the present invention provides the method of providing information, wherein the biological sample contains whole blood, leukocytes, peripheral blood mononuclear cells, buffy coat, plasma, serum, sputum, tears, mucus, nasal washes, nasal aspirate, breath, urine, semen, saliva, peritoneal washings, pelvic fluids, cystic fluid, meningeal fluid, amniotic fluid, glandular fluid, pancreatic fluid, lymph fluid, pleural fluid, nipple aspirate, bronchial aspirate, synovial fluid, joint aspirate, organ secretions, cells, cell extract, or cerebrospinal fluid.

Still yet another embodiment of the present invention provides the method of providing information, wherein measurement of the expression level of the protein or fragment thereof is performed by protein chip assay, immunoassay, ligand-binding assay, MALDI-TOF (matrix-assisted laser desorption/ionization time of flight mass spectrometry) analysis, SELDI-TOF (surface enhanced laser desorption/ionization-time of flight mass spectrometry) analysis, radiation immunoassay, radiation immunodiffusion, Ouchterlony immunodiffusion, rocket immunoelectrophoresis, tissue immunostaining, complement fixation assay, 2D electrophoresis assay, liquid chromatography-mass spectrometry (LC-MS), liquid chromatography-mass spectrometry/mass spectrometry (LC-MS/MS), Western blotting, or enzyme-linked immunosorbent assay (ELISA).

A further embodiment of the present invention provides the method of providing information, wherein measurement of the expression level of the gene encoding the protein or fragment thereof is performed by reverse-transcription polymerase chain reaction (RT-PCR), competitive RT-PCR, real-time RT-PCR, RNase protection assay (RPA), Northern blotting, or DNA chip assay.

Another further embodiment of the present invention provides the method of providing information, wherein the cancer is ovarian cancer, colorectal cancer, pancreatic cancer, gastric cancer, liver cancer, breast cancer, cervical cancer, thyroid cancer, parathyroid cancer, lung cancer, non-small cell lung cancer, prostate cancer, gallbladder cancer, biliary tract cancer, non-Hodgkin's lymphoma, Hodgkin's lymphoma, hematological cancer, bladder cancer, kidney cancer, melanoma, colon cancer, bone cancer, skin cancer, head cancer, uterine cancer, rectal cancer, brain tumor, perianal cancer, fallopian tube carcinoma, endometrial carcinoma, vaginal cancer, vulvar carcinoma, esophageal cancer, small intestine cancer, endocrine adenocarcinoma, adrenal cancer, soft tissue sarcoma, urethral cancer, penile cancer, cancer of ureter, renal cell carcinoma, renal pelvic carcinoma, central nervous system (CNS) tumor, primary CNS lymphoma, spinal cord tumor, brainstem glioma, or pituitary adenoma.

One embodiment of the present invention provides a composition for diagnosing immune-related disease, comprising an agent for measuring the expression level of any one protein or fragment thereof selected from the group consisting of RGS 1, F5, and CD27, or a gene encoding the protein or fragment thereof.

Another embodiment the present invention provides the composition for diagnosing, wherein the protein is a cell surface protein that is expressed specifically on the surface of regulatory T cells.

Still another embodiment of the present invention provides the composition for diagnosing, wherein the regulatory T cells are at least one type selected from the group consisting of effector regulatory T cells, memory regulatory T cells, and regulatory T cells in tumor-infiltrating lymphocytes.

Yet another embodiment of the present invention provides the composition for diagnosing, wherein the agent for measuring the expression level of the protein or fragment thereof comprises at least one selected from the group consisting of an antibody, an oligopeptide, a ligand, a peptide nucleic acid (PNA), and an aptamer, which bind specifically to the protein or fragment thereof.

Still yet another embodiment of the present invention provides the composition for diagnosing, wherein the agent for measuring the expression level of the gene encoding the protein or fragment thereof comprises at least one selected from the group consisting of a primer, a probe, and an antisense oligonucleotide, which bind specifically to the gene.

A further embodiment of the present invention provides the composition for diagnosing, wherein the immune-related disease is autoimmune disease, graft-versus-host disease, organ transplant rejection, asthma, atopy, or acute or chronic inflammatory disease.

Another further embodiment of the present invention provides the composition for diagnosing, wherein the autoimmune disease is selected from the group consisting of rheumatoid arthritis, systemic scleroderma, systemic lupus erythematosus, atopic dermatitis, psoriasis, alopecia areata, asthma, Crohn's disease, Behcet's disease, Sjogren's syndrome, Guillain-Barré syndrome, chronic thyroiditis, multiple sclerosis, multiple myositis, ankylosing spondylitis, fibrositis, and polyarteritis nodosa.

Another embodiment of the present invention provides a kit for diagnosing immune-related disease, comprising the composition for diagnosing.

One embodiment of the present invention provides a method of providing information for diagnosing immune-related disease, comprising a step of measuring the expression level of any one protein or fragment thereof selected from the group consisting of RGS1, F5, and CD27, or a gene encoding the protein or fragment thereof, in a biological sample isolated from a subject.

Another embodiment of the present invention provides the method of providing information, wherein the protein is a cell surface protein that is expressed specifically on the surface of regulatory T cells.

Still another embodiment of the present invention provides the method of providing information, wherein the regulatory T cells are at least one type selected from the group consisting of effector regulatory T cells, memory regulatory T cells, and regulatory T cells in tumor-infiltrating lymphocytes.

Yet another embodiment of the present invention provides the method of providing information, wherein the biological sample contains whole blood, leukocytes, peripheral blood mononuclear cells, buffy coat, plasma, serum, sputum, tears, mucus, nasal washes, nasal aspirate, breath, urine, semen, saliva, peritoneal washings, pelvic fluids, cystic fluid, meningeal fluid, amniotic fluid, glandular fluid, pancreatic fluid, lymph fluid, pleural fluid, nipple aspirate, bronchial aspirate, synovial fluid, joint aspirate, organ secretions, cells, cell extract, or cerebrospinal fluid.

Still yet another embodiment of the present invention provides the method of providing information, wherein measurement of the expression level of the protein or fragment thereof is performed by protein chip assay, immunoassay, ligand-binding assay, MALDI-TOF (matrix-assisted laser desorption/ionization time of flight mass spectrometry) analysis, SELDI-TOF (surface enhanced laser desorption/ionization-time of flight mass spectrometry) analysis, radiation immunoassay, radiation immunodiffusion, Ouchterlony immunodiffusion, rocket immunoelectrophoresis, tissue immunostaining, complement fixation assay, 2D electrophoresis assay, liquid chromatography-mass spectrometry (LC-MS), liquid chromatography-mass spectrometry/mass spectrometry (LC-MS/MS), Western blotting, or enzyme-linked immunosorbent assay (ELISA).

A further embodiment of the present invention provides the method of providing information, wherein measurement of the expression level of the gene encoding the protein or fragment thereof is performed by reverse transcription-polymerase chain reaction (RT-PCR), competitive RT-PCR, real-time RT-PCR, RNase protection assay (RPA), Northern blotting, or DNA chip assay.

Another further embodiment of the present invention provides the method of providing information, wherein the immune-related disease is autoimmune disease, graft-versus-host disease, organ transplant rejection, asthma, atopy, or acute or chronic inflammatory disease.

Still another further embodiment of the present invention provides the method of providing information, wherein the autoimmune disease is selected from the group consisting of rheumatoid arthritis, systemic scleroderma, systemic lupus erythematosus, atopic dermatitis, psoriasis, alopecia areata, asthma, Crohn's disease, Behcet's disease, Sjogren's syndrome, Guillain-Barré syndrome, chronic thyroiditis, multiple sclerosis, multiple myositis, ankylosing spondylitis, fibrositis, and polyarteritis nodosa.

One embodiment of the present invention provides a composition for diagnosing brain nervous system disease, comprising an agent for measuring the expression level of any one protein or fragment thereof selected from the group consisting of RGS1, F5, and CD27, or a gene encoding the protein or fragment thereof.

Another embodiment the present invention provides the composition for diagnosing, wherein the protein is a cell surface protein that is expressed specifically on the surface of regulatory T cells.

Still another embodiment of the present invention provides the composition for diagnosing, wherein the regulatory T cells are at least one type selected from the group consisting of effector regulatory T cells, memory regulatory T cells, and regulatory T cells in tumor-infiltrating lymphocytes.

Yet another embodiment of the present invention provides the composition for diagnosing, wherein the agent for measuring the expression level of the protein or fragment thereof comprises at least one selected from the group consisting of an antibody, an oligopeptide, a ligand, a peptide nucleic acid (PNA), and an aptamer, which bind specifically to the protein or fragment thereof.

Still yet another embodiment of the present invention provides the composition for diagnosing, wherein the agent for measuring the expression level of the gene encoding the protein or fragment thereof comprises at least one selected from the group consisting of a primer, a probe, and an antisense oligonucleotide, which bind specifically to the gene.

A further embodiment of the present invention provides the composition for diagnosing, wherein the brain nervous system disease is a neurodegenerative disease or a neuroinflammatory disease.

Another further embodiment of the present invention provides the composition for diagnosing, wherein the neurodegenerative disease or the neuroinflammatory disease is selected from the group consisting of stroke, dementia, Alzheimer's disease, Parkinson's disease, Huntington's disease, Niemann-Pick disease, multiple sclerosis, prion disease, Creutzfeldt-Jakob disease, frontotemporal dementia, dementia with Lewy bodies, amyotrophic lateral sclerosis, paraneoplastic syndrome, corticobasal degeneration, multiple system atrophy, progressive supranuclear palsy, nervous system autoimmune disease, spinocerebellar ataxia, inflammatory and neuropathic pain, cerebrovascular disease, spinal cord injury, and tauopathy.

Another embodiment of the present invention provides a kit for diagnosing brain nervous system disease, comprising the composition for diagnosing.

One embodiment of the present invention provides a method of providing information for diagnosing brain nervous system disease, comprising a step of measuring the expression level of any one protein or fragment thereof selected from the group consisting of RGS1, F5, and CD27, or a gene encoding the protein or fragment thereof, in a biological sample isolated from a subject.

Another embodiment of the present invention provides the method of providing information, wherein the protein is a cell surface protein that is expressed specifically on the surface of regulatory T cells.

Still another embodiment of the present invention provides the method of providing information, wherein the regulatory T cells are at least one type selected from the group consisting of effector regulatory T cells, memory regulatory T cells, and regulatory T cells in tumor-infiltrating lymphocytes.

Yet another embodiment of the present invention provides the method of providing information, wherein the biological sample contains whole blood, leukocytes, peripheral blood mononuclear cells, buffy coat, plasma, serum, sputum, tears, mucus, nasal washes, nasal aspirate, breath, urine, semen, saliva, peritoneal washings, pelvic fluids, cystic fluid, meningeal fluid, amniotic fluid, glandular fluid, pancreatic fluid, lymph fluid, pleural fluid, nipple aspirate, bronchial aspirate, synovial fluid, joint aspirate, organ secretions, cells, cell extract, or cerebrospinal fluid.

Still yet another embodiment of the present invention provides the method of providing information, wherein measurement of the expression level of the protein or fragment thereof is performed by protein chip assay, immunoassay, ligand-binding assay, MALDI-TOF (matrix-assisted laser desorption/ionization time of flight mass spectrometry) analysis, SELDI-TOF (surface enhanced laser desorption/ionization-time of flight mass spectrometry) analysis, radiation immunoassay, radiation immunodiffusion, Ouchterlony immunodiffusion, rocket immunoelectrophoresis, tissue immunostaining, complement fixation assay, 2D electrophoresis assay, liquid chromatography-mass spectrometry (LC-MS), liquid chromatography-mass spectrometry/mass spectrometry (LC-MS/MS), Western blotting, or enzyme-linked immunosorbent assay (ELISA).

A further embodiment of the present invention provides the method of providing information, wherein measurement of the expression level of the gene encoding the protein or fragment thereof is performed by reverse transcription-polymerase chain reaction (RT-PCR), competitive RT-PCR, real-time RT-PCR, RNase protection assay (RPA), Northern blotting, or DNA chip assay.

Another further embodiment of the present invention provides the method of providing information, wherein the brain nervous system disease is a neurodegenerative disease or a neuroinflammatory disease.

Still another further embodiment of the present invention provides the method of providing information, wherein the neurodegenerative disease or the neuroinflammatory disease is selected from the group consisting of stroke, dementia, Alzheimer's disease, Parkinson's disease, Huntington's disease, Niemann-Pick disease, multiple sclerosis, prion disease, Creutzfeldt-Jakob disease, frontotemporal dementia, dementia with Lewy bodies, amyotrophic lateral sclerosis, paraneoplastic syndrome, corticobasal degeneration, multiple system atrophy, progressive supranuclear palsy, nervous system autoimmune disease, spinocerebellar ataxia, inflammatory and neuropathic pain, cerebrovascular disease, spinal cord injury, and tauopathy.

### Advantageous Effects

The marker protein or fragment thereof specific for regulatory T cells according to the present invention is able to distinguish regulatory T cells from other immune cells, and thus may be used to identify regulatory T cells. In addition, by identifying regulatory T cells expressing the marker specific for regulatory T cells, it is possible to diagnose cancer, immune-related disease, or brain nervous system disease, which is affected by the regulatory T cells.

### Mode for Invention

Hereinafter, the present invention will be described in detail by way of the following examples. However, the following examples are intended only to illustrate the present invention, and the scope of the present invention is not limited by the following examples.

Single cell mRNA sequencing data obtained by analysis from patient samples of a total of three types of cancer (colorectal cancer, hepatocellular carcinoma, and non-small cell lung carcinoma) were applied to a decision-tree-based algorithm called XGBoost (EXtreme Gradient Boosting). At this time, XGBoost found the optimal combination for classification into two sets using the decision-based tree through TPM values. Using this, it was possible to primarily classify which genes are involved in classification and which genes are specifically expressed in tumor Tregs.

### Example 1. Selection of Regulatory T Cell Biomarkers in Colorectal Cancer (CRC)

Patient samples (total of 12 patients = 8 men and 4 women) were collected from peripheral blood, fresh cancer tissue, and normal tissue at least 2 cm from the collected cancer tissue.

Next, single-cell sorting, reverse transcription, amplification and sequencing were performed as follows.
i) A tissue piece of 1 mm³ was cut from each of collected tissues, placed in RPMI-1640 (10% FBS), and enzymatically digested using a MACS tumor dissociation kit (Miltenyl Biotec) in a rotor at 37°C for 30 minutes.
ii) The digested tissue was passed through a 40-µm cell-strainer and centrifuged at 400g for 10 minutes.
iii) The supernatant was discarded, and red blood cells were lysed using RBC lysis buffer for 2 minutes.
iv) After washing twice with PBS, the cell pellet was resuspended in PBS/1% FBS.
v) PBMCs were isolated using HISTOPAQUE-1077.
vi) Staining with human CD3, CD4, CD8, and CD25 antibodies was performed, and single-cell sorting was performed by FACS.
vii) To classify cytotoxic T cells, Th cells, and Treg cells into subsets, the cells were enriched into 7AAD-CD3+CD8+, 7AAD-CD3+CD4+CD25-, and 7AAD-CD3+CD4+CD25hi, respectively, and collected in 96-well plates precooled to 4°C, followed by lysis with lysis buffer.
viii) cDNA was synthesized from the single-cell lysate by reverse transcriptase PCR using the Smart-Seq2 (Nat Protoc. 2014 Jan;9(1):171-81) method, and the mRNA expression level was measured by qPCR.

At this time, the T cell subsets used in analysis were tumor Tregs, peripheral Tregs, Th17, effector memory T cells, and naive T cells. In addition, the number of cells used in analysis was 10,805 cells (data for 12,525 genes) out of 11,138 single cells.

The analysis indicators were as follows:

TPM (transcripts per million) = reads / transcript length / kilobase / 1,000,000

Analysis was performed in the following manner.

The average TPM value for each gene expressed in each subset was calculated:
① Tumor Tregs: 1,319 cells;
② Peripheral Tregs: 365 cells;
③ Th17: 229 cells;
④ Effector memory T cells: 204 cells; and
(5) Naive T cells: 472 cells.

Tumor Treg cells were distinguished from other cell subsets (peripheral Tregs, Th17 cells, effector memory T cells, and naive T cells):
① Tumor Treg cluster: 1,319 single cells; and
② Non-tumor Treg cluster: 1,270 single cells.

The machine learning process was performed using XGBoost as follows:
① TPM values for every single cell were input into XGBoost to generate a conditional algorithm that could most efficiently distinguish tumor Tregs from other T cell subsets. At this time, 474 genes were used in the conditions.
② The algorithm using 474 genes distinguished tumor Tregs from other T cell subsets with an accuracy of 97.5%.

In order to find surface markers among the 474 selected genes, only genes with an expression level of 4 to 5 on the plasma membrane were reselected. In the final step, Treg-specific markers were selected based on the following detailed strategy.
① Scenario 1: Genes whose TPM values in tumor Treg cells are higher than the TPM values in naive T cells
② Scenario 2: Genes whose TPM values in tumor Treg cells are higher than the TPM values in peripheral Treg cells
③ Scenario 3: Genes whose TPM values in peripheral Treg cells are higher than the TPM values in naive T cells
④ Scenario 4: Genes whose TPM values in peripheral Treg cells are higher than the TPM values in Th17 cells

The following markers were selected based on the above scenarios (see Table 1 below):

### Example 2. Selection of Regulatory T Cell Biomarkers in Hepatocellular Carcinoma (HCC)

Patient samples (total of 12 patients = 8 men and 4 women) were collected from peripheral blood, fresh cancer tissue, and normal tissue at least 2 cm from the collected cancer tissue.

Next, single-cell sorting, reverse transcription, amplification and sequencing were performed in the same manner as in Example 1.

However, the T cell subsets used in analysis were tumor Tregs, peripheral Tregs, naive T cells, cytotoxic CD4 cells, and exhausted T cells.

The number of cells used in analysis was 5,063 single cells (data for 23,389 genes).

The analysis indicator and analysis method were performed in the same manner as in Example 1. However, the following cell subsets were used:
① Tumor Tregs: 582 cells;
② Peripheral Tregs: 261 cells;
③ Naive T cells: 646 cells; and
④ Exhausted T cells: 146 cells.

Tumor Treg cells were distinguished from other cell subsets (peripheral Tregs, exhausted T cells, cytotoxic CD4 cells, and naive T cells):
① Tumor Treg cluster: 582 single cells
② Non-tumor Treg cluster: 1,220 single cells

The machine learning process was performed using XGBoost as follows:
① TPM values for every single cell were input into XGBoost to generate a conditional algorithm that could most efficiently distinguish tumor Tregs from other T cell subsets. At this time, 281 genes were used in the conditions.
② The algorithm using 281 genes distinguished tumor Tregs from other T cell subsets with an accuracy of 97.78%.

In order to find surface markers among the selected genes, only genes with an expression level of 4 to 5 on the plasma membrane were reselected. In the final step, Treg-specific markers were selected based on the following detailed strategy.
① Scenario 1: Genes whose TPM values in tumor Treg cells are higher than the TPM values in naive T cells
② Scenario 2: Genes whose TPM values in peripheral Treg cells are higher than the TPM values in naive T cells
③ Scenario 3: Genes whose TPM values in tumor Treg cells are higher than the TPM values in peripheral Treg cells

The following markers were selected based on the above scenarios (see Table 1 below):

### Example 3. Selection of Regulatory T Cell Biomarkers in Non-Small Cell Lung Carcinoma (NSCLC)

Patient samples (11 adenocarcinoma patients, and 3 squamous cell carcinoma patients) were collected from peripheral blood and fresh cancer tissue.

Next, single-cell sorting, reverse transcription, amplification and sequencing were performed in the same manner as in Example 1. However, the T cell subsets used in analysis were tumor Tregs, peripheral Tregs, naive T cells, cytotoxic CD4 cells, exhausted T cells, central memory T cells, and effector memory T cells.

The number of cells used in analysis was 12,346 single cells (data for 12,394 genes).

The analysis indicator and analysis method were performed in the same manner as in Example 1. However, the following cell subsets were used:
① Tumor Tregs: 939 cells;
② Resting Tregs: 428 cells;
③ Naive T cells: 532 cells;
④ Exhausted T cells: 343 cells;
(5) Central memory T cells: 666 cells; and
(6) Effector memory T cells: 434 cells.

Tumor Treg cells were distinguished from other cell subsets (peripheral Tregs, exhausted T cells, cytotoxic CD4 cells, and naive T cells):
① Tumor Treg cluster: 939 single cells
② Non-tumor Treg cluster: 2,398 single cells

The machine learning process was performed using XGBoost as follows:
① TPM values for every single cell were input into XGBoost to generate a conditional algorithm that could most efficiently distinguish tumor Tregs from other T cell subsets. At this time, 548 genes were used in the conditions.
② The algorithm using 548 genes distinguished tumor Tregs from other T cell subsets with an accuracy of 96.71%.

In order to find surface markers among the selected genes, only genes with an expression level of 4 to 5 on the plasma membrane were reselected. In the final step, Treg-specific markers were selected based on the following detailed strategy.
① Scenario 1: Genes whose TPM values in tumor Treg cells are higher than the TPM values in naive T cells
② Scenario 2: Genes whose TPM values in peripheral Treg cells are higher than the TPM values in naive T cells
③ Scenario 3: Genes whose TPM values in tumor Treg cells are higher than the TPM values in peripheral Treg cells

The following markers were selected based on the above scenarios (see Table 1 below):

**[Table 1]**

| **F5** | Treg cells | Peripheral Treg cells | Exhausted T cells | Cytotoxic CD4 cells | Naïve T cells |
|---|---|---|---|---|---|
| CRC | 3.100195011 | -0.399058546 | -0.914322388 | -0.580858528 | |
| HCC | 76.68346154 | 7.718967563 | 13.65846679 | | |
| NSCLC | 2.971520357 | -0.737334345 | -0.384756266 | 0.389661106 | 2.108812156 |
| | | | | | |

| **CD27** | Treg cells | Peripheral Treg cells | Exhausted T cells | Cytotoxic CD4 cells | Naïve T cells |
|---|---|---|---|---|---|
| CRC | 2.499269581 | -2.405700303 | 1.575739572 | 1.529513703 | |
| HCC | 1719.785654 | 516.3749448 | 761.0570309 | | |
| NSCLC | 3.282889886 | -3.151742758 | 1.350902944 | 0.484835081 | 2.482556123 |
| | | | | | |

| **RGS1** | Treg cells | Peripheral Treg cells | Exhausted T cells | Cytotoxic CD4 cells | Naïve T cells |
|---|---|---|---|---|---|
| CRC | 3.517801886 | 3.540307286 | 2.20818689 | -5.350675572 | |
| HCC | 3801.927585 | 5962.456581 | 84.52466424 | | |
| NSCLC | 3.662258478 | -3.686078393 | -3.882425395 | 4.515817357 | -0.340774184 |
| | | | | | |

## Claims

1. A method of identifying regulatory T cells using an antibody or antigen-binding fragment that binds specifically to any one protein or fragment thereof selected from the group consisting of RGS1, F5, and CD27.

2. The method of claim 1, wherein the protein is a cell surface protein that is expressed specifically on surfaces of the regulatory T cells.

3. The method of claim 1, wherein the regulatory T cells are at least one type selected from the group consisting of effector regulatory T cells, memory regulatory T cells, and regulatory T cells in tumor-infiltrating lymphocytes.

4. A composition for diagnosing cancer disease, comprising an agent for measuring an expression level of any one protein or fragment thereof selected from the group consisting of RGS1, F5, and CD27, or a gene encoding the protein or fragment thereof.

5. The composition of claim 4, wherein the protein is a cell surface protein that is expressed specifically on surfaces of regulatory T cells.

6. The composition of claim 5, wherein the regulatory T cells are at least one type selected from the group consisting of effector regulatory T cells, memory regulatory T cells, and regulatory T cells in tumor-infiltrating lymphocytes.

7. The composition of claim 4, wherein the agent for measuring the expression level of the protein or fragment thereof comprises at least one selected from the group consisting of an antibody, an oligopeptide, a ligand, a peptide nucleic acid (PNA), and an aptamer, which bind specifically to the protein or fragment thereof.

8. The composition of claim 4, wherein the agent for measuring the expression level of the gene encoding the protein or fragment thereof comprises at least one selected from the group consisting of a primer, a probe, and an antisense oligonucleotide, which bind specifically to the gene.

9. The composition of any one of claims 4 to 8, wherein the cancer is ovarian cancer, colorectal cancer, pancreatic cancer, gastric cancer, liver cancer, breast cancer, cervical cancer, thyroid cancer, parathyroid cancer, lung cancer, non-small cell lung cancer, prostate cancer, gallbladder cancer, biliary tract cancer, non-Hodgkin's lymphoma, Hodgkin's lymphoma, hematological cancer, bladder cancer, kidney cancer, melanoma, colon cancer, bone cancer, skin cancer, head cancer, uterine cancer, rectal cancer, brain tumor, perianal cancer, fallopian tube carcinoma, endometrial carcinoma, vaginal cancer, vulvar carcinoma, esophageal cancer, small intestine cancer, endocrine adenocarcinoma, adrenal cancer, soft tissue sarcoma, urethral cancer, penile cancer, cancer of ureter, renal cell carcinoma, renal pelvic carcinoma, central nervous system (CNS) tumor, primary CNS lymphoma, spinal cord tumor, brainstem glioma, or pituitary adenoma.

10. A kit for diagnosing cancer disease, comprising the composition of any one of claims 4 to 9.

11. A method of providing information for diagnosing cancer disease, comprising a step of measuring an expression level of any one protein or fragment thereof selected from the group consisting of RGS1, F5, and CD27, or a gene encoding the protein or fragment thereof, in a biological sample isolated from a subject of interest.

12. The method of claim 11, wherein the protein is a cell surface protein that is expressed specifically on surfaces of regulatory T cells.

13. The method of claim 12, wherein the regulatory T cells are at least one type selected from the group consisting of effector regulatory T cells, memory regulatory T cells, and regulatory T cells in tumor-infiltrating lymphocytes.

14. The method of claim 13, wherein the biological sample contains whole blood, leukocytes, peripheral blood mononuclear cells, buffy coat, plasma, serum, sputum, tears, mucus, nasal washes, nasal aspirate, breath, urine, semen, saliva, peritoneal washings, pelvic fluids, cystic fluid, meningeal fluid, amniotic fluid, glandular fluid, pancreatic fluid, lymph fluid, pleural fluid, nipple aspirate, bronchial aspirate, synovial fluid, joint aspirate, organ secretions, cells, cell extract, or cerebrospinal fluid.

15. The method of claim 14, wherein measuring the expression level of the protein or fragment thereof is performed by protein chip assay, immunoassay, ligand-binding assay, MALDI-TOF (matrix-assisted laser desorption/ionization time of flight mass spectrometry) analysis, SELDI-TOF (surface enhanced laser desorption/ionization-time of flight mass spectrometry) analysis, radiation immunoassay, radiation immunodiffusion, Ouchterlony immunodiffusion, rocket immunoelectrophoresis, tissue immunostaining, complement fixation assay, 2D electrophoresis assay, liquid chromatography-mass spectrometry (LC-MS), liquid chromatography-mass spectrometry/mass spectrometry (LC-MS/MS), Western blotting, or enzyme-linked immunosorbent assay (ELISA).

16. The method of claim 14, wherein measuring the expression level of the gene encoding the protein or fragment thereof is performed by reverse transcription-polymerase chain reaction (RT-PCR), competitive RT-PCR, real-time RT-PCR, RNase protection assay (RPA), Northern blotting, or DNA chip assay.

17. The method of any one of claims 11 to 16, wherein the cancer is ovarian cancer, colorectal cancer, pancreatic cancer, gastric cancer, liver cancer, breast cancer, cervical cancer, thyroid cancer, parathyroid cancer, lung cancer, non-small cell lung cancer, prostate cancer, gallbladder cancer, biliary tract cancer, non-Hodgkin's lymphoma, Hodgkin's lymphoma, hematological cancer, bladder cancer, kidney cancer, melanoma, colon cancer, bone cancer, skin cancer, head cancer, uterine cancer, rectal cancer, brain tumor, perianal cancer, fallopian tube carcinoma, endometrial carcinoma, vaginal cancer, vulvar carcinoma, esophageal cancer, small intestine cancer, endocrine adenocarcinoma, adrenal cancer, soft tissue sarcoma, urethral cancer, penile cancer, cancer of ureter, renal cell carcinoma, renal pelvic carcinoma, central nervous system (CNS) tumor, primary CNS lymphoma, spinal cord tumor, brainstem glioma, or pituitary adenoma.

18. A composition for diagnosing immune-related disease, comprising an agent for measuring an expression level of any one protein or fragment thereof selected from the group consisting of RGS1, F5, and CD27, or a gene encoding the protein or fragment thereof.

19. The composition of claim 18, wherein the protein is a cell surface protein that is expressed specifically on surfaces of regulatory T cells.

20. The composition of claim 19, wherein the regulatory T cells are at least one type selected from the group consisting of effector regulatory T cells, memory regulatory T cells, and regulatory T cells in tumor-infiltrating lymphocytes.

21. The composition of claim 20, wherein the agent for measuring the expression level of the protein or fragment thereof comprises at least one selected from the group consisting of an antibody, an oligopeptide, a ligand, a peptide nucleic acid (PNA), and an aptamer, which bind specifically to the protein or fragment thereof.

22. The composition of claim 20, wherein the agent for measuring the expression level of the gene encoding the protein or fragment thereof comprises at least one selected from the group consisting of a primer, a probe, and an antisense oligonucleotide, which bind specifically to the gene.

23. The composition of any one of claims 18 to 22, wherein the immune-related disease is autoimmune disease, graft-versus-host disease, organ transplant rejection, asthma, atopy, or acute or chronic inflammatory disease.

24. The composition of claim 23, wherein the autoimmune disease is selected from the group consisting of rheumatoid arthritis, systemic scleroderma, systemic lupus erythematosus, atopic dermatitis, psoriasis, alopecia areata, asthma, Crohn's disease, Behcet's disease, Sjogren's syndrome, Guillain-Barré syndrome, chronic thyroiditis, multiple sclerosis, multiple myositis, ankylosing spondylitis, fibrositis, and polyarteritis nodosa.

25. A kit for diagnosing immune-related disease, comprising the composition of any one of claims 18 to 24.

26. A method of providing information for diagnosing immune-related disease, comprising a step of measuring the expression level of any one protein or fragment thereof selected from the group consisting of RGS1, F5, and CD27, or a gene encoding the protein or fragment thereof, in a biological sample isolated from a subject of interest.

27. The method of claim 26, wherein the protein is a cell surface protein that is expressed specifically on surfaces of regulatory T cells.

28. The method of claim 27, wherein the regulatory T cells are at least one type selected from the group consisting of effector regulatory T cells, memory regulatory T cells, and regulatory T cells in tumor-infiltrating lymphocytes.

29. The method of claim 28, wherein the biological sample contains whole blood, leukocytes, peripheral blood mononuclear cells, buffy coat, plasma, serum, sputum, tears, mucus, nasal washes, nasal aspirate, breath, urine, semen, saliva, peritoneal washings, pelvic fluids, cystic fluid, meningeal fluid, amniotic fluid, glandular fluid, pancreatic fluid, lymph fluid, pleural fluid, nipple aspirate, bronchial aspirate, synovial fluid, joint aspirate, organ secretions, cells, cell extract, or cerebrospinal fluid.

30. The method of claim 29, wherein measuring the expression level of the protein or fragment thereof is performed by protein chip assay, immunoassay, ligand-binding assay, MALDI-TOF (matrix-assisted laser desorption/ionization time of flight mass spectrometry) analysis, SELDI-TOF (surface enhanced laser desorption/ionization-time of flight mass spectrometry) analysis, radiation immunoassay, radiation immunodiffusion, Ouchterlony immunodiffusion, rocket immunoelectrophoresis, tissue immunostaining, complement fixation assay, 2D electrophoresis assay, liquid chromatography-mass spectrometry (LC-MS), liquid chromatography-mass spectrometry/mass spectrometry (LC-MS/MS), Western blotting, or enzyme-linked immunosorbent assay (ELISA).

31. The method of claim 29, wherein measuring the expression level of the gene encoding the protein or fragment thereof is performed by reverse transcription-polymerase chain reaction (RT-PCR), competitive RT-PCR, real-time RT-PCR, RNase protection assay (RPA), Northern blotting, or DNA chip assay.

32. The method of any one of claims 26 to 31, wherein the immune-related disease is autoimmune disease, graft-versus-host disease, organ transplant rejection, asthma, atopy, or acute or chronic inflammatory disease.

33. The method of 32, wherein the autoimmune disease is selected from the group consisting of rheumatoid arthritis, systemic scleroderma, systemic lupus erythematosus, atopic dermatitis, psoriasis, alopecia areata, asthma, Crohn's disease, Behcet's disease, Sjogren's syndrome, Guillain-Barré syndrome, chronic thyroiditis, multiple sclerosis, multiple myositis, ankylosing spondylitis, fibrositis, and polyarteritis nodosa.

34. A composition for diagnosing brain nervous system disease, comprising an agent for measuring an expression level of any one protein or fragment thereof selected from the group consisting of RGS1, F5, and CD27, or a gene encoding the protein or fragment thereof.

35. The composition of claim 34, wherein the protein is a cell surface protein that is expressed specifically on surfaces of regulatory T cells.

36. The composition of claim 35, wherein the regulatory T cells are at least one type selected from the group consisting of effector regulatory T cells, memory regulatory T cells, and regulatory T cells in tumor-infiltrating lymphocytes.

37. The composition of claim 36, wherein the agent for measuring the expression level of the protein or fragment thereof comprises at least one selected from the group consisting of an antibody, an oligopeptide, a ligand, a peptide nucleic acid (PNA), and an aptamer, which bind specifically to the protein or fragment thereof.

38. The composition of claim 36, wherein the agent for measuring the expression level of the gene encoding the protein or fragment thereof comprises at least one selected from the group consisting of a primer, a probe, and an antisense oligonucleotide, which bind specifically to the gene.

39. The composition of any one of claims 34 to 38, wherein the brain nervous system disease is a neurodegenerative disease or a neuroinflammatory disease.

40. The composition of claim 39, wherein the neurodegenerative disease or the neuroinflammatory disease is selected from the group consisting of stroke, dementia, Alzheimer's disease, Parkinson's disease, Huntington's disease, Niemann-Pick disease, multiple sclerosis, prion disease, Creutzfeldt-Jakob disease, frontotemporal dementia, dementia with Lewy bodies, amyotrophic lateral sclerosis, paraneoplastic syndrome, corticobasal degeneration, multiple system atrophy, progressive supranuclear palsy, nervous system autoimmune disease, spinocerebellar ataxia, inflammatory and neuropathic pain, cerebrovascular disease, spinal cord injury, and tauopathy.

41. A kit for diagnosing brain nervous system disease, comprising the composition of any one of claims 34 to 40.

42. A method of providing information for diagnosing brain nervous system disease, comprising a step of measuring an expression level of any one protein or fragment thereof selected from the group consisting of RGS1, F5, and CD27, or a gene encoding the protein or fragment thereof, in a biological sample isolated from a subject of interest.

43. The method of claim 42, wherein the protein is a cell surface protein that is expressed specifically on surfaces of regulatory T cells.

44. The method of claim 43, wherein the regulatory T cells are at least one type selected from the group consisting of effector regulatory T cells, memory regulatory T cells, and regulatory T cells in tumor-infiltrating lymphocytes.

45. The method of claim 44, wherein the biological sample contains whole blood, leukocytes, peripheral blood mononuclear cells, buffy coat, plasma, serum, sputum, tears, mucus, nasal washes, nasal aspirate, breath, urine, semen, saliva, peritoneal washings, pelvic fluids, cystic fluid, meningeal fluid, amniotic fluid, glandular fluid, pancreatic fluid, lymph fluid, pleural fluid, nipple aspirate, bronchial aspirate, synovial fluid, joint aspirate, organ secretions, cells, cell extract, or cerebrospinal fluid.

46. The method of claim 45, wherein measuring the expression level of the protein or fragment thereof is performed by protein chip assay, immunoassay, ligand-binding assay, MALDI-TOF (matrix-assisted laser desorption/ionization time of flight mass spectrometry) analysis, SELDI-TOF (surface enhanced laser desorption/ionization-time of flight mass spectrometry) analysis, radiation immunoassay, radiation immunodiffusion, Ouchterlony immunodiffusion, rocket immunoelectrophoresis, tissue immunostaining, complement fixation assay, 2D electrophoresis assay, liquid chromatography-mass spectrometry (LC-MS), liquid chromatography-mass spectrometry/mass spectrometry (LC-MS/MS), Western blotting, or enzyme-linked immunosorbent assay (ELISA).

47. The method of claim 45, wherein measuring the expression level of the gene encoding the protein or fragment thereof is performed by reverse transcription-polymerase chain reaction (RT-PCR), competitive RT-PCR, real-time RT-PCR, RNase protection assay (RPA), Northern blotting, or DNA chip assay.

48. The method of any one of claims 42 to 47, wherein the brain nervous system disease is a neurodegenerative disease or a neuroinflammatory disease.

49. The method of claim 48, wherein the neurodegenerative disease or the neuroinflammatory disease is selected from the group consisting of stroke, dementia, Alzheimer's disease, Parkinson's disease, Huntington's disease, Niemann-Pick disease, multiple sclerosis, prion disease, Creutzfeldt-Jakob disease, frontotemporal dementia, dementia with Lewy bodies, amyotrophic lateral sclerosis, paraneoplastic syndrome, corticobasal degeneration, multiple system atrophy, progressive supranuclear palsy, nervous system autoimmune disease, spinocerebellar ataxia, inflammatory and neuropathic pain, cerebrovascular disease, spinal cord injury, and tauopathy.
